# EUROPEAN PATENT APPLICATION

(11) **EP 4 035 693 A1**
(43) Date of publication of application: **03.08.2022**
(21) Application number: 22152054.7
(22) Date of filing: 18.01.2022
(51) Int. Cl.: A61L 2/22, A61L 2/24, A61L 9/14

(54) **SYSTEM, APPARATUS AND METHOD FOR SYNCHRONIZING DOSING EVENTS BETWEEN SPACES**

(30) Priority: 29.01.2021 US 202117162424
(71) Applicant: Christie Digital Systems USA, Inc., Cypress, CA 90630 (US)
(72) Inventor: PECKOVER, Michael Brent, Cypress, 90630 (US)
(74) Representative: Zimmermann, Tankred Klaus

(57) **Abstract**

An example system for synchronizing dosing events between a plurality of spaces includes: a plurality of dosing subsystems, each dosing subsystem corresponding to one of the spaces and configured to administer sanitizing media doses to the corresponding space, wherein each dosing subsystem comprises: a dosing mechanism; and a controller coupled to the dosing mechanism, the controller configured to: obtain a shared dosing schedule, wherein the shared dosing schedule is shared between each of the plurality of dosing subsystems; detect a trigger event for a dosing event in the space; and in response to the trigger event, control the dosing mechanism to administer the sanitizing media in the space in line with the shared dosing schedule. Doses may therefore be scheduled to avoid or minimizes overdoses to humans or other living organisms.

## Description

### FIELD

The specification relates generally to dosing systems, and more particularly to systems, apparatuses and methods for synchronizing dosing events between spaces.

### BACKGROUND

Facilities having multiple spaces and people and/or objects moving through the spaces may desire the spaces to be sanitized regularly to prevent the spread of bacteria and germs, and to maintain a desired or regulated level of cleanliness. The sanitizing media used to sanitize the spaces may be harmful to humans or other living organisms.

### SUMMARY

According to an aspect of the specification, a system for synchronizing dosing events between a plurality of spaces is described. The system includes: a plurality of dosing subsystems, each dosing subsystem corresponding to one of the spaces and configured to administer sanitizing media doses to the corresponding space, wherein each dosing subsystem comprises: a dosing mechanism; and a controller coupled to the dosing mechanism, the controller configured to: obtain a shared dosing schedule, wherein the shared dosing schedule is shared between each of the plurality of dosing subsystems; detect a trigger event for a dosing event in the space; and in response to the trigger event, control the dosing mechanism to administer the sanitizing media in the space in line with the shared dosing schedule.

According to another aspect of the specification, a dosing subsystem is described. The dosing subsystem includes: a dosing mechanism; and a controller coupled to the dosing mechanism, the controller configured to: obtain a shared dosing schedule, wherein the shared dosing schedule is shared between a plurality of dosing subsystems; detect a trigger event for a dosing event in the space; and in response to the trigger event, control the dosing mechanism to administer sanitizing media in the space in line with the shared dosing schedule.

According to another aspect of the specification, a method of synchronizing dosing events between a plurality of spaces is described. The method includes: obtaining, at a dosing subsystem corresponding to one of the spaces, a shared dosing schedule, wherein the shared dosing schedule is shared between dosing subsystems corresponding to each of the plurality of spaces; detecting a trigger event in the space; and in response to the trigger event, administering sanitizing media doses in line with the shared dosing schedule.

### BRIEF DESCRIPTION OF DRAWINGS

Implementations are described with reference to the following figures, in which:
FIG. 1 is a block diagram of an example system for synchronizing dosing events between a plurality of spaces of a facility;
FIG. 2 is a block diagram of certain internal components of a dosing subsystem in the system of FIG. 1;
FIG. 3 is a flowchart of an example method for synchronizing dosing events between a plurality of spaces;
FIG. 4 is a flowchart of an example method of administering doses in line with a shared dosing schedule at block 315 of the method of FIG. 3; and
FIG. 5 is a schematic diagram of an example shared dosing schedule and room dosing schedules.

### DETAILED DESCRIPTION

The management of dose administration in a facility containing multiple spaces can be complicated based on different duty cycles between spaces, triggers occurring at different times and other factors. Some dosing systems may have duty cycles which are scheduled for certain periods in the day, while others are motion triggered. Thus, when a custodian, for example, is moving between spaces, they may receive a first dose based on the scheduled duty cycle, and second, third, and additional doses as they move from one trigger-based space to the next. The repeated doses may be harmful to the health of the person receiving multiple doses. Hence doses may be scheduled or triggered to avoid or minimize overdoses.

An example system minimizes the chances of repeated dosages by aligning the duty cycles and/or dosage schedules for each space with a shared dosing schedule such that the spaces administer their doses at the same time (where possible based on the number and/or frequency of doses to be administered). Thus, rather than a trigger event initiating a dosing event immediately, the system may register the trigger event and administer a dose after a period of time, synchronously with doses administered in other spaces within the facility. The synchronous dose administration reduces the risk of a person receiving multiple doses, since they will only be in one space receiving one dose at a given time.

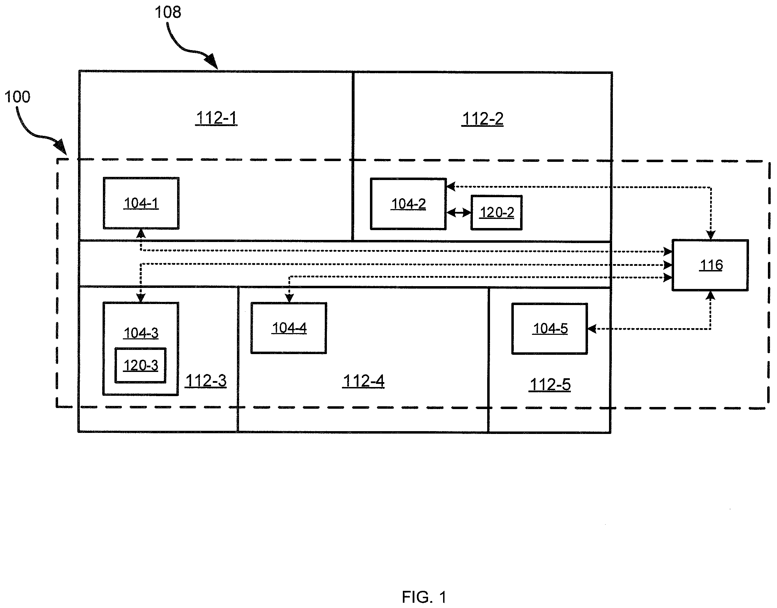

FIG. 1 depicts an example system 100 for synchronizing dosing events between a plurality of spaces. The system 100 includes a plurality of dosing subsystems, of which five examples 104-1, 104-2, 104-3, 104-4, and 104-5 are depicted (referred to herein generically as a dosing subsystem 104 and collectively as the dosing subsystems 104; this nomenclature is used elsewhere herein). The system 100 may be deployed, for example, in a facility 108, such as a hospital, a warehouse, a school, or other public or private buildings. In particular, the facility 108 in which the system 100 is deployed includes a plurality of spaces 112 to be sanitized, of which five examples 112-1, 112-2, 112-3, 112-4, and 112-5 are depicted.

As will be appreciated, in other examples, the system 100 may include more or less than five dosing subsystems 104. More particularly, the number of dosing subsystems 104 forming the system 100 may correspond to the number of spaces 112 to be sanitized. In some examples, a given space 112, more than one dosing subsystem 104 may be employed. Additionally, in other examples, the facility 108 may include spaces which are not to be sanitized by the system 100, and hence the system 100 may correspond only to those spaces 112 of the facility 108 which are to be sanitized.

The dosing subsystems 104 are generally configured to administer doses of a sanitizing media to the given space 112 in which they are deployed to sanitize the space 112. In particular, each dosing subsystem 104 may administer doses of the sanitizing media at the same times as the other dosing subsystems 104 according to a shared dosing schedule, to the extent that individual space dosing schedules permit synchronization. Further, each dosing subsystem 104 may administer doses substantially synchronously to the other dosing subsystems 104 based on the shared dosing schedule and an internal clock, rather than requiring constant communications between each of the dosing subsystems 104. The synchronization of dose administration in different spaces 112 reduces the likelihood that living subjects moving between the spaces 112 of the facility 108 will receive a double dose of the sanitizing media, since the doses are administered at different spaces simultaneously.

In some examples, the system 100 may further include a central control system 116. The central control system 116 may be in communication with each of the dosing subsystems 104, illustrated in the present example by dashed lines. For example, the communication links may include one or more wired or wireless networks, or a combination of wired and wireless networks. The central control system 116 may generally be configured to manage common features, such as tracking a common time to be used at each of the dosing subsystems 104. In some examples, the central control system 116 may further manage the shared dosing schedule to be used by each of the dosing subsystems 104. It will be appreciated that in further examples, the system 100 need not include a central control system.

In some examples, the system 100 may further include one or more sensors to detect events in the spaces 112. In the present example, two sensors 120-2 and 120-3 are depicted to detect the presence of nearby humans in the spaces 112-2 and 112-3, respectively. The sensors 120 may be, for example, optical sensors (e.g., employing infrared light, visible light such as cameras, or the like), accelerometers to detect movement caused by nearby objects or people, audio sensors, or other suitable sensors for detecting nearby humans or other objects. The sensors 120 may be independent components of the system 100 which are interconnected with the corresponding dosing subsystem 104, as illustrated with the sensor 120-2 and the dosing subsystem 104-2. Alternately, the sensors 120 may be integrated with the dosing subsystems 104 themselves, as illustrated with the sensor 120-3 and the dosing subsystem 104-3.

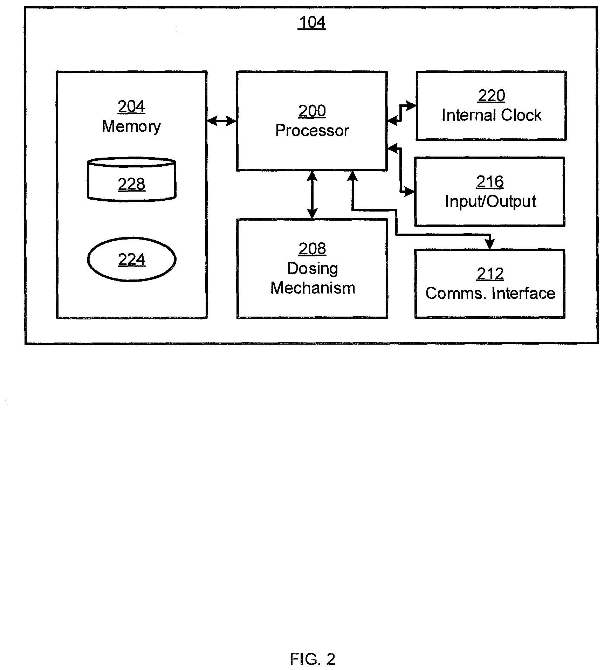

Turning to FIG. 2, an example dosing subsystem 104, including certain internal components, is shown in greater detail. As will be appreciated, each of the dosing subsystems 104-1, 104-2, 104-3, 104-4, and 104-5 may have an internal structure similar to the example dosing subsystem 104 depicted in FIG. 2. The dosing subsystem 104 includes a controller 200, a memory 204 and a dosing mechanism 208. The dosing subsystem 104 may further include a communications interface 212, an input/output device 216, and an internal clock 220.

The controller 200 may be a processor such as a central processing unit (CPU), a microcontroller, a processing core, or similar. The controller 200 may include multiple cooperating processors. In some examples, the functionality implemented by the controller 200 may be implemented by one or more specially designed hardware and firmware components, such as a field-programmable gate array (FPGA), an application-specific integrated circuit (ASIC) and the like. In some examples, the controller 200 may be a special purpose processor which may be implemented via dedicated logic circuitry of an ASIC, an FPGA, or the like in order to enhance the processing speed of the dosing synchronization operation discussed herein.

The controller 200 is interconnected with a non-transitory computer-readable storage medium, such as the memory 204. The memory 204 may include a combination of volatile memory (e.g., random access memory or RAM) and non-volatile memory (e.g., read only memory or ROM, electrically erasable programmable read only memory or EEPROM, flash memory). The controller 200 and the memory 204 may comprise one or more integrated circuits. Some or all of the memory may be integrated with the controller 200. In particular, the memory 204 stores a control application 224 which, when executed by the controller 200, configures the controller 200 to perform various functions discussed below in greater detail and related to the dosing synchronization operation of the system 100. In other examples, the application 224 may be implemented as a suite of distinct applications. The memory 204 may also store a repository 228 configured to store a shared dosing schedule for the dosing synchronization operation, dose scheduling rules to comply with the shared dosing schedule, individual dosing schedules for the given dosing subsystem 104, and the like. In other examples, the memory 204 and/or the repository 228 may also store other rules and data pertaining to the dosing synchronization operation of the system 100.

The dosing mechanism 208 is configured to administer doses of a sanitizing media to the space 112 in which the dosing subsystem 104 is deployed. For example, the dosing mechanism 208 may be a radiation source, for example to irradiate the space 112 with radiation, such as microwaves, infrared waves, gamma rays, ultrasonic rays, electron beams, or other suitable irradiative energy capable of sanitizing the space 112. In other examples, the dosing mechanism 208 may include a supply chamber to contain a supply of the sanitizing media and an aerosol nozzle to dispense the sanitizing media. In such examples, the sanitizing media may be a disinfectant to be dispensed in aerosol form in the space 112. Other sanitizing media will be apparent to those of skill in the art and the dosing mechanism 208 is selected based on the sanitizing media to be administered to the space 112. The dosing mechanism 208 may further be capable of being controlled to adjust the amount (e.g., volume or intensity), as well as the duration and timing of the doses to the space 112. In particular, the dosing mechanism 208 is interconnected with the controller 200 which controls the administration of the doses by the dosing mechanism 208 to the space 112.

The dosing subsystem 104 may further include the communications interface 212 interconnected with the controller 200. The communications interface 212 includes suitable hardware (e.g., transmitters, receivers, network interface controllers and the like) allowing the dosing subsystem 104 to communicate with other computing devices. The specific components of the communications interface 212 are selected based on the type of network or other links that the dosing subsystem 104 is to communicate over. In particular, the communications interface 212 allows the dosing subsystem 104 to communicate with the central control system 116, where necessary, as well as a sensor 120, where applicable.

In some examples, the dosing subsystem 104 may also include one or more input and/or output devices 216. The input/output devices 216 may include one or more buttons, keypads, dials, touch-sensitive display screens, or the like for receiving input from an operator. The output devices may include one or more display screens, sound generators, vibrators, or the like for providing output or feedback to an operator.

The internal clock 220 is a clock to track the time locally at the dosing subsystem 104. In particular, the internal clock 220 allows the dosing subsystem to align with the shared dosing schedule. The dosing subsystem 104 may periodically update the internal clock 220, for example by obtaining a common time tracked by the central control system 116 to ensure that the local time at each of the dosing subsystems 104 is consistent throughout the system 100. For example, the internal clock 220 may be synchronized at predefined intervals (e.g., once per day, once per week, or other suitable intervals), or at each instance that a dose is triggered, or under other suitable conditions. In other examples, the dosing subsystem 104 may obtain the common time from a satellite source or other mutually agreed upon external source.

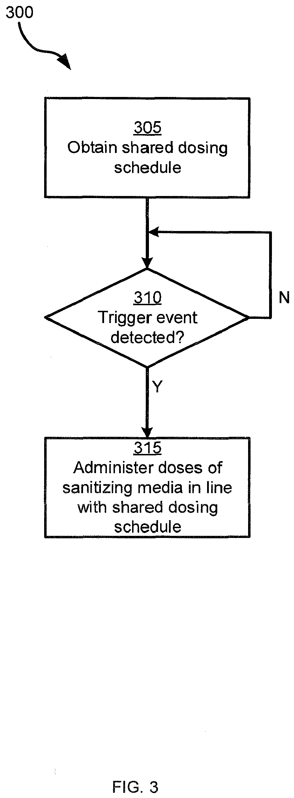

The operation of the system 100 will now be described in greater detail, with reference to FIG. 3. FIG. 3 depicts a flowchart of an example method 300 of synchronizing doses, which will be described in conjunction with its performance in the system 100, with reference to the components illustrated in FIGS. 1 and 2. In particular, the method 300 may be implemented via execution of the application 224 by the controller 200 of a dosing subsystem 104. In other examples, the method 300 may be performed by other suitable devices or in other suitable systems.

At block 305, the controller 200 obtains a shared dosing schedule, wherein the shared dosing schedule is shared between each of the dosing subsystems 104 in the system 100. The shared dosing schedule may, for example, define at least one primary designated time with which respective controllers of each of the dosing subsystems 104 are to align administered doses of the sanitizing media. That is, the primary designated times are times which are to be targeted by the dosing subsystems 104 for administering doses. For example, the primary designated times may be on the hour, at each hour of the day. In some examples, in addition to primary designated times, the shared dosing schedule may additionally include one or more secondary designated times, tertiary designated times, and the like, which are to be targeted by the dosing subsystem 104 for intermediary doses in between doses administered at the primary designated times.

In some examples, the shared dosing schedule may be encoded into the memory 204 of the dosing subsystem 104, for example during manufacture and/or assembly of the dosing subsystems 104. That is, the shared dosing schedule may be fixed amongst all the dosing subsystems 104 manufactured by a given party. Thus, any dosing subsystems 104 acquired from the given party will included a shared dosing schedule which is inherently shared amongst other dosing subsystems. In such examples, at block 305, the controller 200 may simply retrieve the shared dosing schedule from the memory 204.

In other examples, the shared dosing schedule may be dynamically updated, for example, by the central control system 116. In such examples, the controller 200 may request the shared dosing schedule from the central control system 116, for example via the communications interface 212. Upon receipt of the shared dosing schedule, the dosing subsystem 104 may store the shared dosing schedule in the memory 204. At subsequent iterations of the method 300, the controller 200 may retrieve the shared dosing schedule received from the central control system 116 from the memory 204. In some examples, the controller 200 may periodically request updated shared dosing schedules (e.g., after a predefined updated period). In other examples, rather than requesting the shared dosing schedule, the central control system 116 may push the updated shared dosing schedules to the dosing subsystems 104.

In some examples, in addition to obtaining the shared dosing schedule, the controller 200 may additionally obtain the common time, for example from the central control system 116. In other examples, such as those where the system 100 does not include a central control system, the dosing subsystem 104 may obtain the common time from a mutually agreed upon independent source, such as a satellite or the like. After obtaining the common time, the controller 200 may synchronize the internal clock 220 to the common time.

At block 310, the controller 200 determines whether a trigger event is detected. The trigger event may include a power on event, a time-of-day initiation, an external event detected at a sensor (not shown) of the system 100, or similar. Generally, the trigger event represents an event after which sanitization of the space 112 in which the dosing subsystem 104 is to be performed. For example, the dosing subsystem 104 may be configured to periodically administer doses of the sanitizing media whenever the dosing subsystem 104 is operational or powered on, hence the trigger event may be a power on event. In other examples, the dosing subsystem 104 may be configured to administer doses of the sanitizing media between operating hours of the facility 108 (e.g., when people are expected to be moving within the facility 108), and hence the trigger event may be an opening time of the facility 108. In still further examples, the dosing subsystem 104 may be configured to administer doses of the sanitizing media after detection of a nearby person. Accordingly, the system 100 may include a sensor to detect the presence of nearby people. The sensor may be external to but interconnected with the dosing subsystem 104, and hence the trigger event may be an input signal transmitted from the sensor to the dosing subsystem 104. In other examples, the sensor may be integrated with the dosing subsystem 104, and hence the trigger event may be the detection, by the sensor, of a nearby person. As will be appreciated, other trigger events or configurations are also contemplated.

If, at block 310, no trigger event is detected, the dosing subsystem 104 may stand by and continue to wait until a trigger event is detected.

If, at block 310, a trigger event is detected, the method 300 proceeds to block 315. At block 315, the controller 200 is configured to control the dosing mechanism 208 to administer the sanitizing media to the space 112 in line with the shared dosing schedule. For example, the dosing mechanism 208 may administer doses of the sanitizing media to the its respective space 112 at the primary designated times. Specifically, since each of the dosing subsystems 104 follows the same shared dosing schedule and administers doses in line with the primary designated times defined by the shared dosing schedule, each dosing subsystem 104 of the system 100 will administer doses of sanitizing media synchronously.

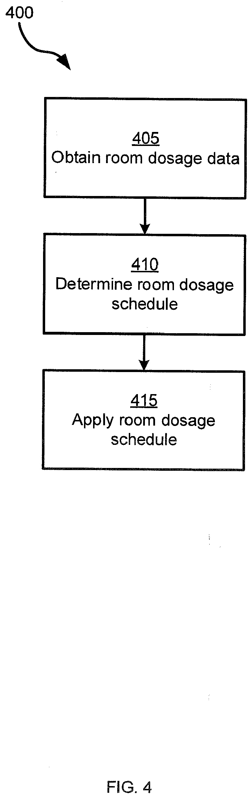

Referring to FIG. 4, a flowchart of an example method 400 of administering doses of the sanitizing media in line with the shared dosing schedule is depicted.

**At** block 405, the controller 200 obtains room dosage data. The room dosage data may be stored for example, in the repository 228 and/or elsewhere in the memory 204. The room dosage data may define parameters for administering doses of the sanitizing media to the space 112. For example, the room dosage data may include a dosage frequency, dosage amount and/or length, and a number of doses to administer or other end parameters. The room dosage data may be expressed, in some examples, as a duty cycle representing a percentage of a period in which the dosing mechanism is to be activated to administer sanitizing media, and may include a prescribed period over which the duty cycle is to be executed. The room dosage data may further include maximum dosage amounts, minimum dosage amounts, and other pertinent dosage data.

At block 410, the controller 200 determines a room dosage schedule based on the room dosage data and the shared dosing schedule. The room dosage schedule defines times at which doses are to be administered, and may additionally specify the quantity (e.g., length and/or amount) of sanitizing media to be administered in the dose. In some examples, the room dosage schedule may be specifically defined by the room dosage data. In other examples, the room dosage schedule may generated based on the dosage amounts, lengths, number of doses, dose intensities, dose durations, and the like specified in the room dosage data. Specifically, the room dosage schedule is defined to administer doses in line with the shared dosing schedule. For example, the times defined in the room dosage schedule may be selected to align with the primary designated times, and, where possible, the secondary, tertiary and other designated times. That is, the room dosage schedule is defined in order to administer the proper amount of sanitizing media to the corresponding space 112 based on the room dosage data, while also defining times at which to administer said doses in order to align the administered doses to the shared dosing schedule, for example by defining doses to be administered at the designated times.

At block 415, the controller 200 applies the room dosage schedule to the dosing mechanism 208 in order to administer the doses of sanitizing media in line with the shared dosing schedule. Specifically, the dosing subsystem 104 may administer doses at the times defined by the room dosage schedule, as coordinated by the common time tracked by the internal clock 220.

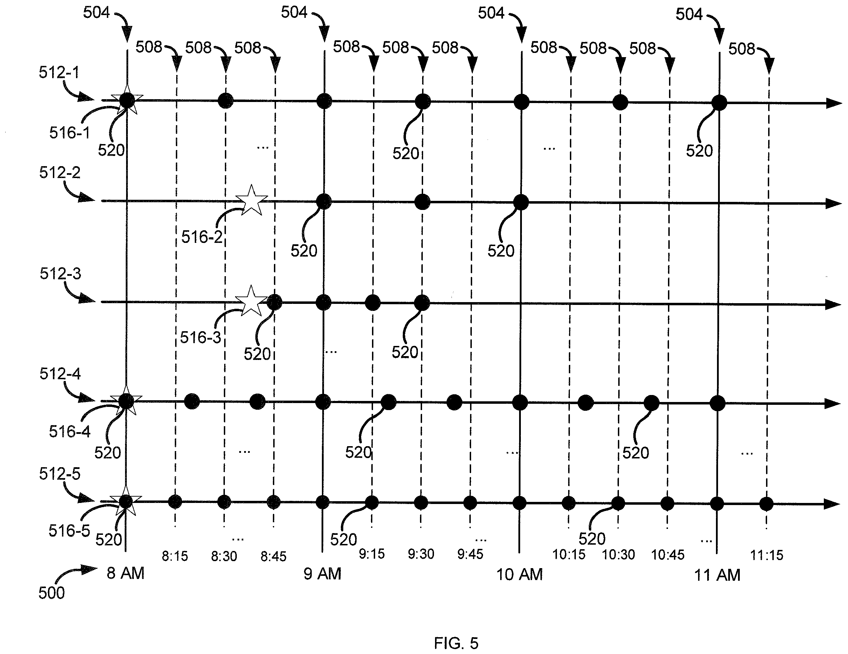

For example, referring to FIG. 5, an example shared dosing schedule 500 is depicted. The shared dosing schedule 500 may include a plurality of primary designated times 504, as well as a plurality of secondary designated times 508. The primary and secondary designated times 504, 508 represent the times at which the dosing subsystems 104 are to target for administering doses of sanitizing media in order to synchronize dose administration. In particular, the dosing subsystems 104 are to first prioritize synchronization with the primary designated times 504, and, where possible, based on the parameters defined in the room dosage data, additionally synchronize doses with the secondary designated times 508.

For example, five example room dosage schedules 512-1, 512-2, 512-3, 512-4, and 512-5 (e.g., for example corresponding to each of the spaces 112) are depicted.

**The** first room dosage schedule 512-1 is initiated when a trigger event 516-1 occurs in the space 112-1. For example, the trigger event 516-1 may correspond to a start time-of-day at which the dosing subsystem 104-1 is to begin sanitizing the space 112-1. In some examples, the room dosage data may include a duty cycle as well as a prescribed period over which the duty cycle is to be executed. For example, the room dosage schedule 512-1 may be determined based on a duty cycle of 5 minutes per 30 minutes, with a prescribed period of 30 minutes. Accordingly, the room dosage schedule 512-1 may be defined with a dose 520 scheduled to be administered every 30 minutes, with the prescribed period of 30 minutes initiated at one of the primary designated times 504 in order to administer the doses 520 in line with the shared dosing schedule. Thus, in the present example, since the trigger event 516-1 aligns with one of the primary designated times 504, one of the doses 520 is administered immediately, at the corresponding primary designated time 504. Additionally, as can be seen, the intermediary doses (i.e., the doses administered between consecutive primary designated times) correspond with secondary designated times 508. The room dosage schedule 512-1 may continue until a defined stop parameter, such as an end time-of-day. Accordingly, the schedule 512-1 defines doses 520 according to the shared dosage schedule between the start time-of-day and the end time-of-day.

The second room dosage schedule 512-2 is initiated when a trigger event 516-2 occurs in the space 112-2. For example, the trigger event 516-2 may correspond to detection of a nearby person. The room dosage data may similarly include a duty cycle and a prescribed period over which the duty cycle is to be executed, as well as a prescribed number of doses. For example, the room dosage schedule 512-2 may be determined based on a duty cycle of 5 minutes per 30 minutes, with a prescribed period of 30 minutes, for 3 doses. Accordingly, the room dosage schedule 512-2 may be defined with a dose 520 scheduled to be administered every 30 minutes, with the prescribed period of 30 minutes corresponding to one of the primary designated times 504 in order to administer the doses 520 in line with the shared dosing schedule. Notably, since the trigger event 516-2 does not align with a primary designated time 504, no doses 520 are administered until the next primary designated time 504 after the trigger event 516-2. Thus, by aligning doses with the shared dosing schedule 500 rather than based solely upon detection of the trigger event 516-2, the dosing subsystem 104-2 may administer doses 520 synchronously with the dosing subsystem 104-1, thereby reducing the likelihood of a person receiving a double dose of the sanitizing media as they move between the spaces 112-1 and 112-2.

The third room dosage schedule 512-3 is initiated when a trigger event 516-3 occurs in the space 112-3. For example, the trigger event 516-3 may also correspond to the detection of a nearby person. The room dosage data may include a duty cycle and a prescribed period over which the duty cycle is to be executed, as well as a prescribed number of doses. For example, the room dosage schedule 512-3 may be determined based on a duty cycle of 3 minutes per 15 minutes, with a prescribed period of 15 minutes, for 4 doses. Accordingly, the room dosage schedule 512-3 may be defined with a dose 520 scheduled to be administered every 15 minutes, with the prescribed period of 15 minutes corresponding to one of the primary designated times 504. In this example, the trigger event 516-3 does not align with a primary designated time 504. Further, the prescribed period of 15 minutes for the duty cycle is less than the time between the trigger event 516-3 and the next primary designated time 504 after the trigger event 516-3. Accordingly, the room dosage schedule 512-3 may define doses 520 both before and after the primary designated time 504 such that at least one of the scheduled doses 520 occurs in line with the primary designated time 504. Thus, by aligning doses with the shared dosing schedule 500 rather than based solely upon detection of the trigger event 516-3, the dosing subsystem 104-3 may administer at least one of the doses 520 synchronously with the dosing subsystem 104-1 and 104-2.

The fourth room dosage schedule 512-4 is initiated when a trigger event 516-4 occurs in the space 112-4. For example, the trigger event 516-4 may correspond to a start time-of-day at which the dosing subsystem 104-4 is to begin sanitizing the space 112-4. In some examples, the prescribed period over which a duty cycle is to be executed may not correspond to the secondary designated times in between the primary designated times. For example, the room dosage schedule 512-4 may be determined based on a duty cycle of 5 minutes per 20 minutes, with a prescribed period of 20 minutes. Accordingly, the room dosage schedule 512-4 may be defined with a dose 520 scheduled to be administered every 20 minutes, as prescribed, with one of the prescribed 20 minute periods initiated at one of the primary designated times 504 in order to administer the doses 520 in line with the shared dosing schedule. Thus, in the present example, since the trigger event 516-4 aligns with one of the primary designated times 504, one of the doses 520 is administered immediately, at the corresponding primary designated time 504. As can be seen, the intermediary doses do not correspond with the secondary designated times 508 but are administered in accordance with the prescribed period defined in the room dosage data. While some of the doses are administered asynchronously as compared to the other dosing subsystems 104, by aligning doses with the shared dosing schedule 500, at least a portion of the doses are still administered synchronously with the other dosing subsystems 104. That is, at least some of the doses administered by the dosing subsystem 104-4 are administered at primary designated times, and hence are delivered synchronously with the dosing subsystems 104-1, 104-2, and 104-3.

A fifth room dosage schedule 512-5 is initiated when a trigger event 516-5 occurs in the space 112-5. For example, the trigger event 516-5 may similarly correspond to a start time-of-day at which the dosing subsystem 104-5 is to begin sanitizing the space 112-5. In some examples, the room dosage data may include a duty cycle without prescribing a specific period over which the duty cycle is to be executed. For example, the room dosage schedule 512-5 may be determined based on a duty cycle of 5 minutes per 20 minutes without specifically prescribing a period of 20 minutes. Accordingly, the dosing subsystem 104-5 may define the duty cycle in terms of a percentage (i.e., on 25% of the time) and may determine a different period for dose administration which better corresponds to the shared dosing schedule. For example, to achieve a 25% duty cycle, the dosing subsystem 104-5 may define an updated duty cycle of 3.75 minutes per 15 minutes, with prescribed periods of 15 minutes. The room dosage schedule 512-5 may therefore be defined to administer begin the period at the primary designated time 504 such that a dose (lasting 3.75 minutes) is initiated at the primary designated time 504 and at each of the secondary designated times 508. Thus, at least some of the doses administered by the dosing subsystem 104-5 are administered synchronously with the other dosing subsystems 104-1, 104-2, 104-3, and 104-4.

It will be appreciated that in other examples, other room dosage data may be provided to the dosing subsystems 104 to generate a room dosage schedule. For example, rather than a duty cycle indicating an amount of time on and off, the room dosage data may define a number of doses. Alternately, the room dosage data may define an amount (e.g., a volume) of aerosol disinfectant to apply, and the dosing subsystems 104 may compute a dosage frequency and a corresponding amount of the aerosol disinfectant to administer in each dose as part of the room dosage schedule. Other possible room dosage data parameters and corresponding room dosage schedule computations will also be apparent to those of skill in the art. Further, in some examples, to synchronize doses between spaces 112, the duty cycles and prescribed periods may be selected to correspond to the shared dosing schedule, where possible.

The scope of the claims should not be limited by the embodiments set forth in the above examples, but should be given the broadest interpretation consistent with the description as a whole.

## Claims

1. A system for synchronizing dosing events between a plurality of spaces, the system comprising:
a plurality of dosing subsystems, each dosing subsystem corresponding to one of the spaces and configured to administer doses of a sanitizing media to the corresponding space, wherein each dosing subsystem comprises:
a dosing mechanism; and
a controller coupled to the dosing mechanism, the controller configured to:
obtain a shared dosing schedule, wherein the shared dosing schedule is shared between each of the plurality of dosing subsystems;
detect a trigger event for a dosing event in the space; and
in response to the trigger event, control the dosing mechanism to administer the sanitizing media in the space in line with the shared dosing schedule.

2. The system of claim 1, wherein the shared dosing schedule includes at least one primary designated time with which respective controllers of each of the dosing subsystems are to align administered doses of the sanitizing media.

3. The system of claim 1, wherein to administer the sanitizing media in line with the shared dosing schedule, each controller is configured to:
obtain room dosage data defining parameters for administering doses to the corresponding space;
determine a room dosage schedule defining times at which the doses are to be administered, the room dosage schedule based on the room dosage data and the shared dosing schedule; and
apply the room dosage schedule to the dosing mechanism.

4. The system of claim 3, wherein the room dosage data includes one or more of: a duty cycle and a prescribed period over which the duty cycle is to be executed.

5. The system of claim 1, wherein each controller is further configured to update a respective internal clock of its respective dosing subsystem according to a common time.

6. The system of claim 1, further comprising a central control system in communication with each of the dosing subsystems, the central control system to manage one or more of: a common time and the shared dosing schedule.

7. The system of claim 1, further comprising a sensor interconnected with a given one of the dosing subsystems, the sensor configured to detect an event in the corresponding space of the given one of the dosing subsystems.

8. A dosing subsystem for dosing a space, the dosing subsystem comprising:
a dosing mechanism; and
a controller coupled to the dosing mechanism, the controller configured to:
obtain a shared dosing schedule, wherein the shared dosing schedule is shared between a plurality of dosing subsystems;
detect a trigger event for a dosing event in the space; and
in response to the trigger event, control the dosing mechanism to administer sanitizing media in the space in line with the shared dosing schedule.

9. The dosing subsystem of claim 8, wherein the trigger event comprises one or more of: a power on event, a time-of-day initiation, and an external event detected at a sensor.

10. The dosing subsystem of claim 8, wherein the sanitizing media comprises one or more of: an aerosol disinfectant, and radiation.

11. The dosing subsystem of claim 8, wherein to administer the sanitizing media in line with the shared dosing schedule, the controller is configured to:
obtain room dosage data defining parameters for administering doses to the space;
determine a room dosage defining times at which the doses are to be administered, the room dosage schedule based on the room dosage data and the shared dosing schedule; and
apply the room dosage schedule to the dosing mechanism.

12. The dosing subsystem of claim 8, further comprising an internal clock to track a common time.

13. The dosing subsystem of claim 12, wherein the controller is configured to periodically update the internal clock from one of: a central control system tracking the common time, and a satellite source.

14. The dosing subsystem of claim 8, wherein to obtain the shared dosing schedule, the controller is configured to:
(i) retrieve the shared dosing schedule from a memory of the dosing subsystem; or
(ii) receive the shared dosing schedule from a central control system.

15. The dosing subsystem of claim 8, further comprising a sensor to detect the trigger event.

16. A method of synchronizing dosing events between a plurality of spaces, the method comprising:
obtaining, at a dosing subsystem corresponding to one of the spaces, a shared dosing schedule, wherein the shared dosing schedule is shared between dosing subsystems corresponding to each of the plurality of spaces;
detecting a trigger event in the space; and
in response to the trigger event, administering sanitizing media doses in line with the shared dosing schedule.

17. The method of claim 16, wherein administering the sanitizing media doses in line with the shared dosing schedule comprises:
obtaining room dosage data for the space;
determining a room dosage schedule based on the room dosage data and the shared dosing schedule; and
applying the room dosage schedule to a dosing mechanism to administer the sanitizing media doses.

18. The method of claim 17, wherein the room dosage schedule defines times at which the sanitizing media doses are to be administered, and wherein the times are selected to correspond to designated times of the shared dosing schedule.
